(19) Europäisches Patentamt
European Patent Office
Office européen des brevets

(11) Numéro de publication: **0 039 653**
**B1**

# (12) FASCICULE DE BREVET EUROPEEN

(45) Date de publication du fascicule du brevet: **29.06.83**

(51) Int. Cl.³: **C 07 C 51/12**, C 07 C 53/08

(21) Numéro de dépôt: **81420067.1**

(22) Date de dépôt: **04.05.81**

## (54) Préparation de l'acide acétique par carbonylation.

(30) Priorité: **06.05.80 FR 8010541**

(43) Date de publication de la demande:
**11.11.81 Bulletin 81/45**

(45) Mention de la délivrance du brevet:
**29.06.83 Bulletin 83/26**

(84) Etats contractants désignés:
**AT BE CH DE FR GB IT LI LU NL SE**

(56) Documents cités:
**EP - A - 0 010 295**

(73) Titulaire: **RHONE-POULENC CHIMIE DE BASE**
**25, quai Paul Doumer**
**F-92408 Courbevoie (FR)**

(72) Inventeur: **Gauthier-Lafaye, Jean**
**21, rue Duguesclin**
**F-69006 Lyon (FR)**
Inventeur: **Perron, Robert**
**La Pecolière**
**F-69390 Charly (FR)**

(74) Mandataire: **Varniere-Grange, Monique et al,**
**RHONE-POULENC RECHERCHES Centre de Recherches de Saint-Fons Service Brevets B.P. 62**
**F-69190 Saint-Fons (FR)**

**EP 0 039 653 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Préparation de l'acide acétique par carbonylation

La présente invention a pour objet un procédé de préparation de l'acide acétique, par carbonylation du méthanol.

Il est bien connu, en particulier, que l'acide acétique peut être produit par carbonylation du méthanol, dans des conditions relativement sévères de pression, en présence de nickel et d'un halogène libre ou combiné.

C'est ainsi qu'il a éte proposé, notamment, (Cf Brevet des ETATS UNIS D'AMERIQUE n° 2 729 651) de réaliser la carbonylation du méthanol en présence de complexes du nickel, obtenus par réaction d'halogénures de nickel avec des halogénures d'ammonium (ou de phosphonium) quaternaire, complexes de formule générale:

$$[A_4M]_2NiX_4$$

dans laquelle X représente un atome de brome ou d'iode, M un atome de phosphore ou d'azote, A étant, par exemple, un radical alcoyle inférieur.

Ces complexes peuvent être engagés sous cette forme dans la réaction en cause, ou bien ils peuvent être formés in situ. Toutefois, bien que la pression mise en oeuvre lors de la réaction de carbonylation soit élévé (de l'ordre de 700 atmosphères) l'efficacité du système catalytique, exprimée en terme de productivité horaire, est très faible.

Cette productivité exprimée soit par rapport au volume réactionnel, soit par rapport à la masse de nickel mise en oeuvre, a pu être sensiblement améliorée en engageant dans la réaction en cause d'une part, un halogénure de nickel et d'autre part, un halogénure d'ammonium (ou de phosphonium) quaternaire en quantité supérieure à celle requise par la stoechiométrie de formation des complexes de formule rappelée ci-avant. (Cf. Brevet allemand 933.148). Toutefois, même dans ce dernier cas, les conditions de pression restent sévères.

Plus récemment, il été proposé des systèmes catalytiques permettant de carbonyler le méthanol dans des conditions moins sévères de pression. Ainsi, la demande de brevet français 2.370.023 décrit la carbonylation du méthanol en présence de nickel, d'une phosphine libre et/ou complexée avec le nickel, et d'au moins 10 moles d'iodure de méthyle pour 100 moles de méthanol, sous une pression inférieure à 70 bars. Néanmoins, l'efficacité d'un tel système, exprimée en terme de productivité horaire reste faible.

Dans le cadre d'un procédé de carbonylation sous faible pression, il a également été souligné (cf. demande de brevet français 2.404.618) que la présence de solvants, tels que des acides carboxyliques ou leurs esters, a un effet favorable sur le déroulement de la réaction de carbonylation. Néanmoins, l'utilisation de tels solvants n'est pas essentielle et l'alcool dont on part peut servir de solvant.

Toutefois, l'interêt industriel de ces techniques récentes est compromis par l'instabilité des phosphines ou des amines nécessaires à leur mise en oeuvre.

Il a maintenant été trouvé qu'il est possible de préparer des acides carboxyliques, en particulier des acides alcanoïques inférieurs et notamment l'acide acétique par carbonylation d'alcools en présence de nickel et d'au moins un promoteur halogéné avec une productivité remarquable, dans des conditions de pression relativement douces, tout en obviant aux inconvénients précités.

La présente invention a donc pour objet un procédé perfectionné de carbonylation du méthanol en phase liquide à une température supérieure à 120°C et sous une pression totale inférieure à 200 bars en présence d'une quantité efficace de nickel, d'un halogénure d'alkyle ou d'acyle, d'au moins un sel de lanthanide et d'un acide carboxylique initialement chargé.

Il a été trouvé de manière inattendue, en particulier dans le cadre de la carbonylation du méthanol en acide acétique, que les résultats ne sont pas satisfaisants si la pression partielle du monoxyde de carbone est trop élevée; ceci est d'autant plus surprenant que de nombreux travaux antérieurs avaient mis en évidence la nécessité de travailler sous des pressions très élevées.

Les recherches qui ont abouti à la présente invention ont permis de faire ressortir le comportement surprenant des sels de lanthanides comme cocatalyseurs de la réaction de carbonylation d'un alcool, en phase liquide et sous une pression totale inférieure à 200 bars. La demanderesse a en effet constaté que les sels et autres dérivés de nombreux métaux (y compris des métaux carbonyles) ne permettent pas de faire réagir dans les conditions précitées le monoxyde de carbone sur un alcool, dans un acide carboxylique, en présence de nickel et d'un promoteur halogéné.

Le procédé selon la présente invention est conduit en phase liquide en présence d'un acide carboxylique, de formule $R^1$—COOH, initialement chargé, dans lequel $R^1$ représente un radical alkyle linéaire, ramifie ou cyclique renfermant de 1 à 6 atomes de carbone ou un radical $\emptyset$—$C_nH_{2n}$— dans lequel n est un entier compris entre 1 et 6 ($1 \leq n \leq 6$). En d'autres termes l'acide carboxylique qui joue en quelque sorte un rôle de solvant n'est pas forcément l'acide carboxylique produit par la réaction de carbonylation. Toutefois, il peut s'avérer préférable que l'acide carboxylique utilisé comme solvant soit celui produit par la réaction. Néanmoins, l'usage à titre de solvent d'un acide carboxylique plus lourd que l'acide produit, peut faciliter les opérations de séparation.

L'acide carboxylique $R^1$COOH représente au moins 10% en volume du milieu réactionnel initial.

De préférence, il représente au moins 20% en volume du milieu réactionnel. Il peut représenter une partie importante du milieu réactionnel, notamment dans le cas d'une opération réalisée en continu en injectant l'alcool dans le réacteur de carbonylation.

Le procédé selon l'invention nécessite la présence d'une quantité efficace de nickel. N'importe quelle source de nickel peut être mise en oeuvre dans le cadre du présent procédé. On peut introduire le nickel sous sa forme métallique (nickel RANEY, par exemple) ou sous toute autre forme commode. A titre d'exemples de composés du nickel susceptibles d'être utilisés pour la mise en oeuvre du présent procédé, on peut citer: le carbonate, l'oxyde, l'hydroxyde, les halogénures, en particulier l'iodure, les carboxylates, en particulier l'acétate, de nickel. Le nickel carbonyle convient également bien. On utilise de préférence le nickel RANEY, l'iodure de nickel, l'acétate de nickel et le nickel carbonyle.

La quantité de nickel n'est pas critique. La teneur en nickel qui a une influence sur la vitesse de réaction est déterminée en fonction de la vitesse de réaction que l'on estime convenable. De manière générale, une quantité comprise entre 5 et 2000 milliatomes-grammes de nickel par litre de solution conduit à des résultats satisfaisants. On opère de préférence avec une teneur comprise entre 20 et 1000 milliatomes-grammes de nickel par litre.

Le procédé selon la présente invention exige également la présence d'au moins un halogénure d'alkyle ou d'acyle. Ces halogénures ont respectivement pour formule

$$R^2X \text{ et } R^2—\underset{\underset{\displaystyle O}{\|}}{C}—X$$

dans lesquelles X représente un atome de chlore, de brome ou, de préférence, un atome d'iode, et $R^2$ a la signification donnée pour $R^1$, $R^2$ et $R^1$ pouvant être identiques ou différents. Bien entendu l'halogénure d'alkyle qui peut être engagé initialement dans le milieu réactionnel, est susceptible d'être formé in situ à partir de dérivés halogénés tels que $Cl_2$, $Br_2$, $I_2$, HCl, HBr, HI, $NiBr_2$ et $NiI_2$, et de l'alcool (matière de départ). En d'autres termes une partie ou la totalité de l'halogénure d'alkyle nécessaire à la mise en oeuvre du présent procédé peut être formé à partir de ses "précurseurs" définis ci-avant.

On constatera en outre que lorsque le dérivé halogéné est choisi parmi les composés du nickel, il peut être considéré comme précurseur non seulement de l'halogénure d'alkyle mais encore comme précurseur du catalyseur métallique. Dans ce cas particulier il s'avère préférable de charger en outre, initialement, un halogénure d'alkyle ou d'acyle et/ou un précurseur distinct des halogénures de nickel en cause.

Dans le cadre de la présente invention, les iodures d'alkyles inférieurs ayant de 1 à 4 atomes de carbone, constituent une classe préférée d'halogénures d'alkyles. L'iodure de méthyle convient particulièrement bien à la mise en oeuvre du procédé selon l'invention.

Pour une bonne mise en oeuvre du présent procédé une concentration en halogénure d'alkyle ou d'acyle d'au moins 0,5 mole par litre de milieu réactionnel est généralement requise. Bien que l'augmentation de la concentration en halogénure d'alkyle ou d'acyle ait un effet favorable sur la vitesse de réaction, il s'avère préférable de ne pas dépasser une concentration de l'ordre de 8 moles par litre. A ce titre une concentration en halogénure d'alkyle ou d'acyle comprise entre 0,8 et 6 moles/litre et de préférence entre 1,5 et 5 moles/litre conduit à des résultats satisfaisants.

L'une des caractéristiques essentielles de la présente invention réside dans la mise en oeuvre d'au moins un sel de lanthanide, c'est à dire d'au moins un composé choisi parmi les halogénures, les hydroxydes, les oxychlorures, les carbonates, les oxalates et les carboxylates des éléments dont le numéro atomique est compris entre 57 inclu et 71 inclu; les anions carboxylates peuvent être représentés par la formule $R^3—COO^-$, dans laquelle $R^3$ a la signification donnée pour $R^1$, $R^3$ et $R^1$ pouvant être identiques ou différents; les sels de lanthanides peuvent être sous forme hydratée. Les sels de lanthane, de cérium et de néodyme conviennent particulièrement bien à la mise en oeuvre du présent procédé.

La nature précise de la partie anionique du sel de lanthanide ne paraît pas être un paramètre fondamental du présent procédé. Les carboxylates de lanthanide et plus particulièrement les acétates sont d'un emploi commode et à ce titre peuvent être préconisés pour la mise en oeuvre de la présente invention. Les acétates de lanthane, de cérium et de néodyme conviennent bien.

De bons résultats sont obtenus lorsque le rapport atomique M/Ni, M désignant un lanthanide, est compris entre 0,1 et 200 bien, que des rapports inférieurs ou supérieurs puissent être choisis. Ce rapport est avantageusement fixé à une valeur comprise entre 0,5 et 100, et, de préférence, entre 1 et 50.

Une température de réaction d'au moins 120°C est généralement requise pour obtenir une vitesse de réaction satisfaisante. Une gamme de température comprise entre 160 et 220°C s'avère avantageuse.

Dans le cadre du présent procédé il n'est pas nécessaire de purifier ou sécher l'alcool et l'acide carboxylique engagés initialement à la réaction. On peut utiliser des alcools et des acides carboxyliques de qualité technique, renfermant éventuellement jusqu'à 20% en volume d'eau. D'autre part on peut engager à la réaction tout ou partie de l'alcool sous forme d'un ester d'un acide $R^1COOH$ $R^1$ ayant la

signification donnée précédemment. Dans ce cas on devra également engager initialelement de l'eau en quantité au moins égale à la quantité théoriquement nécessaire pour hydrolyser l'ester chargé initialement.

Le procédé de carbonylation selon la présente invention est conduit en phase liquide sous une pression supérieure à la pression atmosphérique, la pression étant toutefois inférieure à 200 bars. On recommande plus particulièrement d'opérer sous une pression partielle de monoxyde de carbone comprise entre 10 et 100 bars. On met en oeuvre, de préférence, du monoxyde de carbone sous forme essentiellement pure, tel qu'il se présente dans le commerce. Toutefois la présence d'impuretés telles que, par exemple du dioxyde de carbone, de l'oxygène, du méthane et de l'azote n'est pas nuisible. La présence d'hydrogène n'est pas nuisible, même en des proportions relativement importantes.

En fin de réaction, on sépare le mélange réactionnel en ses divers constituants par tout moyen approprié, par exemple par distillation.

Le procédé selon l'invention convient particulièrement bien à la préparation de l'acide acétique par carbonylation du méthanol, notamment dans l'acide acétique. Les exemples ci-après illustrent la présente invention sans toutefois en limiter le domaine ou l'esprit.

Dans les exemples les conventions suivantes ont été utilisées:

—AcOH désigne l'acide acétique

—AcOMe désigne l'acétate de méthyle

—RR désigne le rapport molaire

$$\frac{(AcOH+AcOMe)\text{ dose} - (AcOH)\text{ initial}}{(CH_3OH+CH_3I)\text{ initial}}$$

c'est-à-dire le rapport molaire entre "l'acide acétique potentiel" formé et $(CH_3OH+CH_3I)$ initial.

—t désigne la durée effective de l'absorption du monoxyde de carbone à la température de l'essai

—T désigne la durée de l'essai en température.

—Pr désigne la productivité, ramenée au temps t (exprimé en heures), en gramme "d'acide acétique potentiel" formé par litre du mélange réactionnel initial

## Exemple 1

Dans une autoclave en Hastelloy B 2, de 125 ml de capacité on charge:

—372 mMol de méthanol soit 15 ml

—360 mMol d'acide acétique soit 20 ml

—202 mMol d'iodure de méthyle soit 12,5 ml

—40 mAt-g de lanthane sous forme d'acétate de lanthane, soit 12,64 g

—20 mMol d'acétate de nickel tétrahydraté, soit 5,0 g

Après fermeture de l'autoclave on établit une pression de 40 bars d'oxyde de carbone. L'agitation par une système de va et vient est mise en route et l'autoclave est porté à 180°C, en 20 minutes environ, au moyen d'un four annulaire. La pression dans l'autoclave est alors de 66 bars; elle est ensuite maintenue égale à 70 bars par des recharges de CO pur.

L'absorption d'oxyde de carbone est terminée après 2 heures de réaction à 180°C; le chauffage est néanmoins poursuivi pendant encore 5 minutes à cette température.

Après quoi l'agitation et le chauffage sont arrêtés; l'autoclave est refroidi et dégazé.

Le mélange réactionnel résultant est analysé par chromatographie gazeuse, après dilution. Il contient 51,4 g d'acide acétique (RR=87%) et 0,30 d'acétate de méthyle.

La productivité (Pr) de la réaction en acide acétique a donc été de 300 grammes par heure et par litre (g/h x 1).

## Exemples 2 à 6

On a reproduit l'exemple 1 ci-avant mais en utilisant divers cocatalyseurs. Les conditions particulières ainsi que les résultats obtenus figurent dans le tableau I ci-après.

*Essais témoins (a) à (l):*

Dans l'appareillage et selon le mode opératoire décrits pour l'exemple 1 on réalise une série d'essais sur une charge comprenant 15 ml de méthanol, 20 ml d'acide acétique et de l'iodure de méthyle à 180°C et sous 70 bars en présence de divers composés métalliques. *Aucune réaction n'a été observée après 2 heures à la température mentionnée.*

Les conditions particulières de ces essais témoins figurent dans le tableau II ci-après.

TABLEAU I

| N° Ex | NICKEL nature | NICKEL mAt-g | COCATALYSEUR nature | COCATALYSEUR mAt-g | T | t | AcOH (g) | AcOMe (g) | RR (%) | Pr g/hxl |
|---|---|---|---|---|---|---|---|---|---|---|
| 1 | $Ni(OAc)_2$ | 20 | $La(OAc)_3$ | 40 | 2h 05 mn | 2h | 51,4 | 0,30 | 87 | 300 |
| 2 | " | " | $Ce(OAc)_3$ | " | 2h 07 mn | 2h | 58,0 | 0 | 100 | 370 |
| 3 | " | " | $Nd(OAc)_3$ | " | 1h 55 mn | 1h 50 mn | 54,7 | 0 | 97,5 | 350 |
| 4 | " | " | $Eu(OA)_3$ | " | 2h 10 mn | 2h 10 mn | 47,6 | 0,81 | 70 | 240 |
| 5 | " | " | $Yb(OAc)_3$ | " | 3h | 2h 45 mn | 52,6 | 0 | 91 | 230 |
| 6 | $Ni(CO)_4$ | " | $Ce(OAc)_3$ | 100 | 2h | 2h | 25,8 | 8,81 | 37 | ND |

NB: dans ce tableau on a désigné par Ni $(OAc)_2$ l'acétate de nickel tétrahydraté.
ND: non déterminé.

TABLEAU II
Essais témoins

| REF | NICKEL Nature | NICKEL mAt-g | $CH_3I$ en mMol | COMPOSE METALLIQUE Nature | COMPOSE METALLIQUE MAt-g |
|---|---|---|---|---|---|
| a | $Ni(CO)_4$ | 20 | 201 | $Cr(CO)_6$ | 50 |
| b | $Ni(CO)_4$ | 20 | 206 | $Zn(OAc)_2,2H_2O$ | 100 |
| c | $Ni(CO)_4$ | 20 | 203 | Zn | 50 |
| d | $Ni(CO)_4$ | 20 | 209 | $MN(OAc)_2,4H_2O$ | 100 |
| e | $Ni(CO)_4$ | 20 | 204 | $AlI_3$ | 50 |
| f | $Ni(CO)_4$ | 20 | 203 | $Co(OAc)_2,4H_2O$ | 100 |
| g | $Ni(OAc)_2,4H_2O$ | 10 | 205 | $MoBr_3$ | 30 |
| h | $Ni(OAc)_2,4H_2O$ | 8 | 202 | $Fe(OAc)_2$ | 100 |
| i | $Ni(CO)_4$ | 20 | 205 | $MoI_3$ | 10 |
| j | $Ni(CO)_4$ | 10 | 209 | $WI_3$ | 10 |
| k | $Ni(CO)_4$ | 20 | 211 | $Mc(OAc)_2,4H_2O$ | 100 |
| l | $Ni(CO)_4$ | 8 | 200 | $BiI_3$ | 40 |

**Revendications**

1. Procédé de préparation de l'acide acétique par réaction du monoxyde de carbone sur le méthanol en présence d'une quantité efficace de nickel, d'un halogénure d'alkyle ou d'acyle, d'un acide carboxylique initialement chargé, en phase liquide, à une température supérieure à 120°C et sous une pression totale inférieure à 200 bars, caractérisé en ce que la réaction est conduite en présence d'au moins un sel de lanthanide.

2. Procédé selon la revendication 1, caractérisé en ce que l'halogènure d'alkyle ou d'acyle est un iodure.

3. Procédé selon la revendication 2, caractérisé en ce que l'halogénure d'alkyle est un iodure d'alkyle en $C_1$—$C_4$.

4. Procédé selon la revendication 3, caractérisé en ce que l'iodure d'alkyle est l'iodure de méthyle.

5. Procédé selon l'une quelconque des revendications précédentes, caractérisé en ce que l'acide carboxylique initialement chargé a pour formule $R^1$—COOH dans laquelle $R^1$ représente un radical alkyle linéaire, ramifié ou cyclique, en $C_1$—$C_8$ ou un radical $\emptyset$—$C_nH_{2n}$—, $1 \leq n \leq 6$.

6. Procédé selon la revendication 5, caractérisé en ce que l'acide carboxylique initialement chargé est l'acide acétique.

7. Procédé selon l'une quelconque des revendications précédentes, caractérisé en ce que le sel de lanthanide est choisi parmi les halogénures, les hydroxydes, les oxychlorures, les carbonates, les oxalates et les carboxylates des éléments de numéro atomique 57 à 71, les anions carboxylates ayant pour formule $R^3$—$COO^-$ dans laquelle $R^3$ a la signification donnée pour $R^1$ dans la revendication 5, $R^3$ et $R^1$ pouvant être identiques ou différents.

8. Procédé selon la revendication 7, caractérisé en ce que le cation du sel est choisi parmi les cations du lanthane, du cérium et du néodyme.

9. Procédé selon la revendication 7 ou 8, caractérisé en ce que la partie anionique du sel est un carboxylate de formule $R^3$—$COO^-$, dans laquelle $R^3$ a la signification donnée pour $R^1$ dans la revendication 5, $R^3$ et $R^1$ pouvant être identiques ou différents.

10. Procédé selon la revendication 9, caractérisé en ce que la partie anionique du sel est un acétate.

11. Procédé selon l'une quelconque des revendications 7 à 10, caractérisé en ce que le rapport atomique M/Ni, M désignant un lanthanide, est compris entre 0,1 et 200 et, de préférence, entre 0,5 et 100.

12. Procédé selon la revendication 11, caractérisé en ce que le rapport M/Ni est compris entre 1 et 50.

13. Procédé selon l'une quelconque des revendications précédentes, caractérisé en ce que la température de réaction est comprise entre 160 et 220°C.

14. Procédé selon l'une quelconque des revendications précédentes, caractérisé en ce que la pression partielle du monoxyde de carbone est comprise entre 10 et 100 bars.

**Claims**

1. Process for the preparation of acetic acid by reacting carbon monoxide with methanol in the presence of an effective amount of nickel, an alkyl or acyl halide and a carboxylic acid introduced initially, in the liquid phase, at a temperature above 120°C and under a total pressure of less than 200 bars, characterised in that the reaction is carried out in the presence of at least one lanthanide salt.

2. Process according to Claim 1, characterised in that the alkyl or acyl halide is an iodide.

3. Process according to Claim 2, characterised in that the alkyl halide is a $C_1$—$C_4$ alkyl iodide.

4. Process according to Claim 3, characterised in that the alkyl iodide is methyl iodide.

5. Process according to any one of the preceding claims, characterised in that the carboxylic acid introduced initially has the formula $R^1$—COOH, in which $R^1$ represents a linear, branched or cyclic $C_1$—$C_8$ alkyl radical or a radical $\emptyset$—$C_nH_{2n}$—, $1 \leq n \leq 6$.

6. Process according to Claim 5, characterised in that the carboxylic acid introduced initially is acetic acid.

7. Process according to any one of the preceding claims, characterised in that the lanthanide salt is chosen from amongst the halides, the hydroxides, the oxychlorides, the carbonates, the oxalates and the carboxylates of the elements having an atomic number of 57 to 71, the carboxylate anions having the formula $R^3$—$COO^-$, in which $R^3$ has the meaning given for $R^1$ in Claim 5, it being possible for $R^3$ and $R^1$ to be identical or different.

8. Process according to Claim 7, characterised in that the cation of the salt is chosen from amongst the cations of lanthanum, cerium and neodymium.

9. Process according to Claim 7 or 8, characterised in that the anionic part of the salt is a carboxylate of the formula $R^3$—$COO^-$, in which $R^3$ has the meaning given for $R^1$ in Claim 5, it being possible for $R^3$ and $R^1$ to be identical or different.

10. Process according to Claim 9, characterised in that the anionic part of the salt is an acetate.

11. Process according to any one of Claims 7 to 10, characterised in that the atomic ratio M/Ni, M denoting a lanthanide, is between 0.1 and 200 and preferably between 0.5 and 100.

12. Process according to Claim 11, characterised in that the ratio M/Ni is between 1 and 50.

13. Process according to any one of the preceding claims, characterised in that the reaction temperature is between 160 and 220°C.

14. Process according to any one of the preceding claims, characterised in that the partial pressure of the carbon monoxide is between 10 and 100 bars.

**Patentansprüche**

1. Verfahren zur Herstellung von Essigsäure durch Umsetzung von Kohlenmonoxid mit Methanol in Gegenwart einer wirksamen Menge Nickel, eines Alkyl- oder Acyl-halogenids und einer vorgelegten Carbonsäure, in flüssiger Phase bei einer Temperatur oberhalb 120°C und unter einem Gesamtdruck von weniger als 200 bar, dadurch gekennzeichnet, daß die Umsetzung in Gegenwart mindestens eines Salzes (bzw. Hydroxids) von Lanthan oder einem Lanthanid durchgeführt wird.

2. Verfahren nach Anspruch 1, dadurch gekennzeichnet, daß das Alkyl- oder Acyl-halogenic ein Iodid ist.

3. Verfahren nach Anspruch 2, dadurch gekennzeichnet, daß das Alkyl-halogenid ein $C_1$—$C_4$-Alkyliodid ist.

4. Verfahren nach Anspruch 3, dadurch gekennzeichnet, daß das Alkyliodid Methyliodid ist.

5. Verfahren nach einem der vorangehenden Ansprüche, dadurch gekennzeichnet, daß die vorgelegte Carbonsäure der Formel $R^1COOH$ entspricht, in der $R^1$ eine lineare, verzweigte oder cyclische $C_1$—$C_6$-Alkylgruppe oder eine Gruppe $C_6H_5$—$C_nH_{2n}$— mit $1 \leqq n \leqq 6$ bedeutet.

6. Verfahren nach Anspruch 5, dadurch gekennzeichnet, daß die vorgelegte Carbonsäure Essigsäure ist.

7. Verfahren nach einem der vorangehenden Ansprüche, dadurch gekennzeichnet, daß Salz (bzw. Hydroxid) von Lanthan oder einem Lanthanid ausgewählt wird unter den Halogeniden, Hydroxiden, Oxychloriden, Carbonaten, Oxalaten und carbonsauren Salzen der Elemente mit Ordnungszahl 57 bis 71, wobei die Carbonsäureanionen der Formel $R^3$—COO— entsprechen, in der $R^3$ die in Anspruch 5 für $R^1$ angegebene Bedeutung hat und $R^3$ und $R^1$ gleich oder verschieden sein können.

8. Verfahren nach Anspruch 7, dadurch gekennzeichnet, daß das Kation des Salzes ausgewählt wird unter den Kationen von Lanthan, Cer und Neodym.

9. Verfahren nach Anspruch 7 oder 8, dadurch gekennzeichnet, daß das Anion des Salzes ein Carbonsäureanion der Formel $R^3$—$COO^-$ ist, in der $R^3$ die für $R^1$ im Anspruch 5 angegebene Bedeutung hat, wobei $R^3$ und $R^1$ gleich oder verschieden sein können.

10. Verfahren nach Anspruch 9, dadurch gekennzeichnet, daß das Anion des Salzes das Acetatanion ist.

11. Verfahren nach einem der Ansprüche 7 bis 10, dadurch gekennzeichnet, daß das Atomverhältnis M/Ni, wobei M Lanthan oder ein Lanthanid bedeutet, 0,1 bis 200 und vorzugsweise 0,5 bis 100 beträgt.

12. Verfahren nach Anspruch 11, dadurch gekennzeichnet, daß das Verhältnis M/Ni 1 bis 50 beträgt.

13. Verfahren nach einem der vorangehenden Ansprüche, dadurch gekennzeichnet, daß die Reaktionstemperatur im Bereich von 160 bis 200°C liegt.

14. Verfahren nach einem der vorangehenden Ansprüche, dadurch gekennzeichnet, daß der Partialdruck des Kohlenmonoxids im Bereich von 10 bis 100 bar liegt.